# FASCICULE DE BREVET EUROPEEN

(11) **EP 0 620 747 B1**
(45) Date de publication et mention de la délivrance du brevet: **11.03.1998**
(21) Numéro de dépôt: 92923039.9
(22) Date de dépôt: 13.10.1992
(51) Int. Cl.: A61M 5/148

(54) **APPAREIL AUTONOME DE PERFUSION**
UNABHÄNGIGE INFUSIONSVORRICHTUNG
SELF-CONTAINED DRIP APPARATUS

(43) Date de publication de la demande: 26.10.1994
(73) Titulaire: E.R.I.A., 1931 Luxembourg (LU)
(72) Inventeur: PICLIN, Jean-Jacques, F-54123 Viterne (FR); THIBAUT, Gilbert, F-54000 Nancy (FR); BOISSON, Michel, F-59350 Saint-André (FR)
(74) Mandataire: Van Malderen, Michel
(86) Numéro de dépôt international: FR9200960
(87) Numéro de publication internationale: WO9408645

(56) Documents cités:
- EP-A- 0 289 361
- DE-C- 4 129 271
- FR-A- 1 107 676
- FR-A- 2 677 887
- US-A- 4 157 771
- US-A- 4 504 267

## Description

La présente invention a pour objet un appareil autonome pour l'injection à un patient par perfusion d'un liquide contenu dans une poche de perfusion selon le préambule de la revendication.

On décrira dans ce qui suit une application à la perfusion humaine. On comprendra que le dispositif de l'invention peut également trouver une application dans le domaine vétérinaire.

Le problème posé consiste à injecter à un patient, en général mais non limitativement une personne victime d'un accident de la route ou autre, un liquide par perfusion, en général un liquide biologique permettant de compenser les différents pertes en eau du patient en général en état de choc.

De manière habituelle, le liquide est contenu dans une poche de perfusion suspendue à une potence, ou maintenue à la main au moins temporairement par le personnel soignant, de laquelle il s'écoule par simple effet de gravité.

Si cette configuration est parfaitement opérationnelle en milieu hospitalier, il n'en va pas de même lors d'interventions d'urgence, en particulier sur le terrain au moment par exemple d'accidents de la route ou autres.

A cette fin on a donc déjà proposé un certain nombre d'appareils autonomes de perfusion, directement liés à un membre du patient (bras ou jambe) ou suspendu à celui-ci par un moyen quelconque (agrafe de ceinture, etc...).

Aucun des dispositifs connus ne donne entière satisfaction.

Ainsi dans le document FR 2 561 923 on propose un dispositif de ce type dans lequel le liquide de perfusion est expulsé d'une poche par un piston actionné par un ressort.

Ce dispositif présente l'inconvénient de faire appel à des poches de perfusion non standard et d'opérer sur la poche un effort de pression qui n'est pas constant et n'autorise pas un débit régulier du liquide de perfusion.

De même, le document FR 2 570 949 décrit un dispositif de même type pour poche de perfusion standard, dans lequel l'expulsion du liquide intervient par pression d'air comprimé la poche étant disposée dans une chambre étanche. Ces dispositifs n'ont pas pour l'instant pu être mis en oeuvre valablement, en raison de problèmes d'étanchéité et de régulation du débit du gaz de pression.

Ils nécessitent en outre un détendeur et sont donc relativement onéreux.

Le document US-A-4 504 267 décrit un appareil selon le préambule de la revendication. Un tel appareil comprend quatre ressorts agissant directement sur un plateau mobile de telle sorte que pour obtenir une pression constante dans la poche de perfusion, la poche doit être disposée et centrée correctement sur le plateau mobile.

L'invention se propose de remédier à ces différents inconvénients des dispositifs de l'art antérieur en permettant :
- l'utilisation de poches de perfusion standard sous emballage souple stérile ;
- la fixation de l'appareil sur la cuisse ou l'avant-bras du patient ; et fonctionnant sous les conditions suivantes :
   - manipulation par une personne seule ;
   - fonctionnement autonome après mise en place ;
   - temps de perfusion réglable pour une dose standard ;
   - faible prix de revient ;
   - coût de maintenance pendant le stockage le plus faible possible ;
   - légèreté ;
   - fiabilité absolue.
- débit constant à partir de la poche pleine jusqu'à ce qu'elle soit totalement vidée.

Conformément à l'invention, ce résultat est obtenu avec un appareil autonome pour l'injection à un patient par perfusion d'un liquide contenu dans une poche de perfusion tel que spécifié dans la revendication.

On comprendra mieux l'invention à l'aide de la description faite ci-après d'un mode de mise en oeuvre donné à titre d'exemple non limitatif, en référence aux dessins annexés dans lesquels :
- la figure 1 est une vue du dispositif de l'invention en élévation latérale, couvercle ouvert ;
- la figure 2 est une vue en perspective du moyen de sollicitation élastique du plateau.

Le dispositif de perfusion, généralement référencé (1), comporte essentiellement :
- un boitier (2),
- un plateau mobile (3),
- un couvercle (4) articulé sur le boitier (2) en (5),
- un mécanisme d'expulsion (6).

On n'a pas représenté les éléments liés à l'injection du produit.

En fonctionnement, on dispose la poche de perfusion sur le plateau et on referme le couvercle (4) par la poignée (7). Ce simple mouvement arme le mécanisme (6) qui, grâce à des moyens élastiques tend à repousser le plateau vers le haut pour expulser le liquide à perfuser hors de la poche, prise en sandwich entre le plateau et le couvercle.

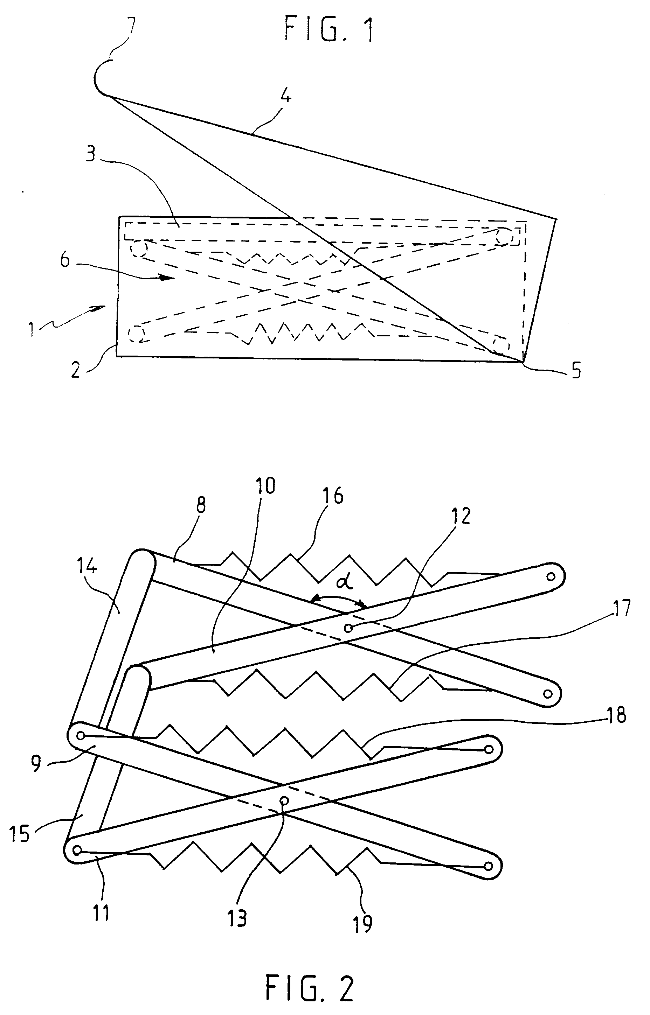

On décrira maintenant le mécanisme (6), en référence à la figure 2.

Le mécanisme est composé de quatre bras (8,10;9,11) articulés deux à deux en leur milieu en (12,13) pour former deux équerres.

Chaque équerre est reliée à l'autre de manière symétrique par des rouleaux (14,15) mobiles en rotation, disposés aux extrémités respectivement des bras parallèles (8,9;10,11).

Les autres extrémités des bras parallèles (8,9;10,11) sont reliées respectivement au boitier (2) et au plateau (3).

Les bras (8,10;9,11) de chaque équerre sont reliés par deux ressorts (16,17;18,19) disposés respectivement de chaque côté des articulations (12,13).

On appellera α l'angle entre les deux bras de chaque équerre. Par symétrie, on notera que l'angle α a la même valeur pour les deux équerres.

Le fonctionnement est le suivant.

Lorsque la pochette est pleine sur le plateau mobile et que le couvercle est rabattu le plateau mobile descend en bandant les ressorts et en augmentant l'angle α. Lorsque le plateau mobile remonte la pochette se vide, la force des ressorts diminue ainsi que l'angle α.

En définissant judicieusement la force des ressorts, l'angle d'origine et de fin de course du plateau mobile et la longeur des bras, la force résultante s'appliquant sur le plateau mobile permet d'obtenir une pression constante dans la pochette et donc un débit constant.

Ce dispositif peut trouver une application également dans d'autres domaines non médicaux, par exemple pour le domaine alimentaire, ou l'injection d'eau dans les plantes pour leur arrosage.

## Revendications

1. Appareil autonome pour l'injection à un patient par perfusion d'un liquide contenu dans une poche de perfusion, ledit dispositif comportant des moyens pour expulser le liquide hors de ladite poche et pour l'injecter au patient, ledit moyen d'expulsion consistant en un plateau (3) sur lequel est disposée la poche de perfusion, le plateau (3) étant disposé dans un boîtier (2) muni d'un couvercle (4) rabattable sur le plateau (3), le plateau (3) étant sollicité par un mécanisme (6) armé par la fermeture du couvercle (4) et tendant à repousser le plateau (3) vers le couvercle (4) en vue d'expulser le liquide hors de la poche de perfusion, caractérisé en ce que ledit mécanisme (6) est composé de quatre bras (8,10;9,11) articulés deux à deux en leur milieu en (12,13) pour former deux équerres, chaque équerre étant reliée à l'autre de manière symétrique par des rouleaux (14,15) mobiles en rotation, disposés aux extrémités respectivement des bras parallèles (8,9;10,11), les autres extrémités desdits bras parallèles (8,9;10,11) étant reliées respectivement au boîtier (2) et au plateau (3), et que les bras (8,10;9,11) de chaque équerre sont reliés par deux ressorts (16,17;18,19) disposés respectivement de chaque côté des articulations (12,13).

## Claims

1. Autonomous device for injecting to a patient through perfusion a liquid contained in a perfusion pouch, said device comprising means for expelling said liquid from said pouch and injecting it to the patient, said expelling means consisting in a plate (3) on which the perfusion pouch is arranged, said plate (3) being situated in a housing (2) provided with a cover (4) which can be folded back to said plate (3), said plate (3) being acted upon by a mechanism (6) being set by closing said cover (4) and tending to force the plate (3) towards the cover (4) so as to expel the liquid out of the perfusion pouch, characterized in that said mechanism (6) is formed with four arms (8, 10; 9, 11) hinged two by two in their center at (12, 13) so as to form two squares, each square being connected with each other symmetrically through rotating rolls (14, 15), arranged at the ends, respectively, of the parallel arms (8, 9; 10, 11), the other ends of said parallel arms (8, 9; 10, 11) being connected, respectively, with the housing (2) and the plate (3), and the arms (8, 10; 9, 11) of each square are connected by two springs (16, 17; 18, 19) arranged, respectively, on each side of the hinges (12, 13).

## Patentansprüche

1. Autonomer Apparat, um einem Patienten durch Infusion eine in einem Infusionsbeutel enthaltene Flüssigkeit zu injizieren, umfassend Mittel, um die Flüssigkeit aus dem Beutel auszutreiben und dem Patienten zu injizieren, wobei die Austreibungsmittel aus einer Platte (3) bestehen, auf der der Infusionsbeutel angeordnet ist, die Platte (3) in einem Gehäuse (2) angeordnet ist, das mit einem auf die Platte (3) herabklappbaren Deckel (4) versehen ist, die Platte (3) durch einen Mechanismus (6) belastet wird, der durch Schließen des Deckels (4) gespannt wird, und bestrebt ist, die Platte (3) zu dem Deckel (4) hin zu schieben, um die Flüssigkeit aus dem Infusionsbeutel auszutreiben, dadurch gekennzeichnet, daß der Mechanismus (6) aus vier Armen (8, 10; 9, 11) besteht, die in ihrer Mitte bei (12, 13) paarweise gelenkig verbunden sind, um zwei Winkelhaken zu bilden, wobei jeder Winkelhaken durch drehbare Rollen (14, 15), die an den Enden der parallelen Arme (8, 9; 10, 11) angeordnet sind, auf symmetrische Weise mit dem anderen verbunden ist, und die anderen Enden der parallelen Arme (8, 9; 10, 11) mit dem Gehäuse (2) bzw. der Platte (3) verbunden sind, und daß die Arme (8, 10; 9, 11) jedes Winkelhakens durch zwei Federn (16, 17; 18, 19), die auf jeder Seite der Gelenke (12, 13) angeordnet sind, verbunden sind.
